# EUROPEAN PATENT APPLICATION

(11) **EP 2 163 646 A1**
(43) Date of publication of application: **17.03.2010**
(21) Application number: 08015582.3
(22) Date of filing: 04.09.2008
(51) Int. Cl.: C12Q 1/68

(54) **CpG island sequencing**

(71) Applicant: Roche Diagnostics GmbH, 68305 Mannheim (DE); F. Hoffmann-La Roche AG, 4070 Basel (CH); Universität Duisburg-Essen, 45141 Essen (DE); Universität Bielefeld, 33501 Bielefeld (DE)
(72) Inventor: Frey, Bruno, 82377 Penzberg (DE); Betzl, Gisela, 82346 Andechs (DE); Fritzilias, Epameinondas, 33602 Bielefeld (DE); Rahmann, Sven, 44139 Dortmund (DE); Horsthemke, Bernhard, 46147 Oberhausen (DE); Zeschnigk, Michael, 42699 Solingen (DE)
(74) Representative: Hildebrandt, Martin K. E.

(57) **Abstract**

The present invention is directed to a method for analyzing the methylation status of a genomic DNA sample, comprising the steps of (i) fragmenting said sample and enriching said sample for sequences comprising CpG islands, (ii) generating a single stranded DNA library, (iii) subjecting said sample to Bisulfite treatment, (iv) clonally amplifying individual members of said single stranded DNA library by means of emulsion PCR, and (v) sequencing said amplified clonally amplified single stranded DNA library.

## Description

The present invention relates to the field of analysis of the methylation status of genomic DNA. More precisely, the present invention provides a new method for analyzing the methylation status of a genome by means of performing conventional Bisulfite treatment of genomic DNA followed by high throughput sequencing.

### Background of the Invention

It is understood in the art that epigenetics in general and DNA methylation at its C residues in particular plays an important role in the regulation of gene expression. But whereas the genetic information itself, contained in the DNA sequence of a gene, is quite fixed, its expression can be strongly varying.

In the mammalian genome, methylation takes place predominantly at cytosine bases in CpG dinucleotide. While this dinucleotide is generally underrepresented in the whole genome, short genomic sequences exist, that are rich in the CpG dinucleotides and are known as CpG islands. These regions are frequently associated with gene promoters and are made up of a few hundred or more nucleotides (Gardiner-Garden, M., and Frommer, M., CpG islands in vertebrate genomes, J Mol Biol 196 (1987) 261-282; Takai, D., and Jones, P.A., Comprehensive analysis of CpG islands in human chromosomes 21 and 22, Proc Natl Acad Sci USA 99 (2002) 3740-3745). Hypomethylation or complete absence of methylation of CpG islands is typically associated with the activity of genes; in contrast hypermethylation of CpG islands within promoter regions has been correlated with decreased gene activity (Jaenisch, R., and Bird, A., Epigenetic regulation of gene expression: how the genome integrates intrinsic and environmental signals, Nature Genetics Supplement 33 (2003) 245-254).

Thus, DNA methylation plays an important role in the epigenetic regulation of gene activity. Methylation patterns are established in early development and are stably propagated during cell proliferation. CpG islands (CpGs) are regarded as key elements in epigenetic gene regulation. With the exception of CpGs associated with imprinted genes or with genes located on the X chromosome these regions are usually maintained free of methylation. Altered methylation is associated with various human diseases and aging. Alterations in DNA methylation patterns are among the earliest and most common events in tumor genesis (Baylin, S.B., Esteller, M., Rountree, M.R., Bachman, K.E., Schuebel, K., Herman, J.G., Aberrant, patterns of DNA methylation, chromatin formation and gene expression in cancer, Hum Mol Genet 10 (2001) 687-692).

Lister, R., et al., Cell 133 (2008) 523-536 have disclosed a shotgun whole genome approach for analyzing the methylation status of multiple genomic regions by means of Bisulfite treatment and subsequent sequencing. However, the disclosed method does not allow for selective enrichment and subsequent sequence analysis of CpG island sequences in particular.

Thus it was an object of the present invention to provide an improved method for whole genome and in particular CpG island methylation status analysis.

### Summary of the Invention

The present invention is directed to a method for analyzing the methylation status of a genomic DNA sample, comprising the steps of
a) fragmenting said sample and enriching said sample for sequences comprising CpG islands
b) generating a single stranded DNA library
c) subjecting said sample to Bisulfite treatment
d) clonally amplifying individual members of said single stranded DNA library by means of emulsion PCR
e) sequencing said clonally amplified single stranded DNA library.

In particular, the methylation status of at least 1000 and preferably at least 5000 CpG islands is analyzed simultaneously.

In one embodiment, step a) comprises the steps of
- digesting said sample with a plurality of restriction enzymes which more frequently cut regions of DNA comprising no CpG islands and less frequently cut regions comprising CpG islands
- isolating DNA fragments with a specific size range.

In one embodiment, said size range has a lower limit of 100, preferably 200 and most preferably 300 base pairs and an upper limit of 1000, preferably 900 and most preferably 800 base pairs.

Said restriction enzymes can be selected from a group consisting of MseI, Tsp509, AluI, NlaIII, BfaI, HpyCH4, DpuI , MboII, MlyI, and BCCI, and any isoschizomer thereof.

In a specific embodiment, step b) comprises the steps of
- performing a fragment polishing reaction
- performing a ligation reaction comprising two different double stranded adaptors A and B, and
- selecting for single stranded molecules comprising one adaptor A and one adaptor B.

The fragment polishing reaction can be performed in the presence of 5-Methyl-dCTP. Similarly, the C-residues of adaptors A and B can be 5-Methyl-C residues.

Furthermore, between said steps of performing a ligation reaction and selecting for single stranded molecules comprising one adaptor A and one adaptor B, a nick repair / fill-in reaction can be performed in the presence of 5-Methyl-dCTP.

### Detailed Description of the Invention

Recently, several methodologies for large scale sequence analysis have become available. According to the present invention, this possibility is exploited in order to perform high resolution methylation analysis on several thousand CpGs in genomic DNA. Therefore, the present invention is directed to a method for analyzing the methylation status of a genomic DNA sample, comprising the steps of
a) fragmenting said sample and enriching said sample for sequences comprising CpG islands
b) generating a single stranded DNA library
c) subjecting said sample to Bisulfite treatment
d) clonally amplifying individual members of said single stranded DNA library by means of emulsion PCR
e) sequencing said clonally amplified single stranded DNA library.

In other words, the present invention allows for analyzing the methylation status of all regions of a complete genome, where changes in methylation status are expected to have an influence on gene expression. Due to the combination of bisulfite treatment, clonal amplification and high throughput sequencing, it is possible to analyze the methylation status of at least 1000 and preferably 5000 CpG islands in parallel.

According to the present invention the step of enriching the sample for sequences comprising CpG islands can be done in different ways. A first possibility for enrichment is immunoprecipitation of methylated DNA using a methyl-Cytosine specific antibody (Weber, M., et al., Nature Genetics 37 (2005) 853-862).

Alternatively, the enrichment part of step a) comprises the steps of
- digesting said sample with a plurality of restriction enzymes which more frequently cut regions of DNA comprising no CpG islands and less frequently cut regions comprising CpG islands, and
- isolating DNA fragments with a specific size range.

Such restrictions enzymes can be selected by a person skilled in the art using conventional Bioinformatics approaches. The selection of appropriate enzymes also has a substantial influence on the average size of fragments that ultimately will be generated and sequenced. The selection of appropriate enzymes may be designed in such a way that it promotes enrichment of a certain fragment length. Thus, the selection may be adjusted to the kind of sequencing method which is finally applied. For most sequencing methods, a fragment length between 100 and 1000 bp has been proven to be highly efficient. Therefore, in one embodiment, said size range has a lower limit of 100, preferably 200 and most preferably 300 base pairs and an upper limit of 1000, preferably 900 and most preferably 800 base pairs.

The human genome reference sequence (NCBI Build 36.1 from March 2006; assembled parts of chromosomes only) has a length of 3,142,044,949 bp and contains 26,567 annotated CpG islands (CpGs) for a total length of 21,073,737 bp (0.67%). In the context of the present invention it is defined that a DNA sequence read hits a CpG if the read overlaps with the CpG by at least 50bp. Assuming an average read length of 100nt (454 GS20 system) and assuming that reads are randomly sampled from the genome, a fraction of 0.67% is expected of all reads to hit a CpG. Since the methylation status of CpGs is of primary interest, randomly sampling reads would waste more than 99% of reads. Thus, the first goal is therefore to prepare genomic samples with an enriched proportion of CpGs.

According to the present invention, the overall strategy is to use an appropriate combination of restriction enzymes and gel-based fragment length selection to ensure that fragments selected from a suitable length range frequently hit CpGs. To ensure physical sequenceability of the fragments and good coverage of the selected genomic region, the fragment length is constrained by 300nt from below and 800nt from above, and the fact that we will accommodate at most 100000 different fragments per haploid genome.

The following enzymes or their isoschizomers (with the following restriction sites) can be used for a method according to the present invention: MseI (TTAA), Tsp509 (AATT), AluI (AGCT), NlaIII (CATG), BfaI (CTAG), HpyCH4 (TGCA), DpuI (GATC), MboII (GAAGA), MlyI (GAGTC), BCCI (CCATC). Isoschizomers are pairs of restriction enzymes specific to the same recognition sequence and cut in the same location. To ensure short fragments in AT-rich regions, the presence of MseI and Tsp509 is required. To keep the experimental protocol simple, preferably at most 3 of the remaining 8 enzymes for 1+8+28+56 = 93 different combinations are chosen.

To select an enzyme combination and a length range that lead to a fragment set with optimally enriched CpG content, extensive in-silico experiments were performed by computationally digesting the human reference genome, and examined various properties of the resulting set of in-silico fragments (ISFs) for each enzyme combination: In the context of the present invention it is defined that an ISF *hits* a CpG if at least 50 of 110 bp of the ISF at either end overlap with a CpG.

For each ISF, three different items recorded:
- whether an ISF in the length range hits a CpG at both ISF ends, at a single end, or at none of its ends (leading to a CpG score of 2, 1, or 0 for that ISF). The sum of CpG scores for all ISFs is a quantity to be maximized with the enzyme combination
- the same score for overlaps with annotated ALU repeats in the genome, to be minimized;
- the number of distinct CpGs hit by the ISFs, to be maximized.

Further statistics were computed to assess the robustness of the length range selection

All 93 enzyme combinations {MseI (TTAA), Tsp509 (AATT)} plus up to three out of {AluI (AGCT), NlaIII (CATG), BfaI (CTAG), HpyCH4 (TGCA), DpuI (GATC), MboII (GAAGA), MlyI (GAGTC), BCCI (CCATC)}, were applied to in-silico digest the human genome reference sequence (NCBI Build 36.1). Even though fragments with sticky ends are obtained in vitro, for the in-silico experiment the cut site was placed in the middle of the recognizing sequence for palindromic sequences, and at the right (3') end for non-palindromic sequences. This introduces only a small length difference for the predicted in-silico fragments (ISFs). The maximal ISF length was set to 800 and the minimal length was set such that approximately 100000 distinct ISFs were obtained. The resulting fragment set for each enzyme combination was evaluated according to the following criteria:

A CpG score indicating how many ISF ends hit a CpG; a hit occurred when at least 50bp of the exterior 110bp of an ISF overlapped with a CpG.

The number of fragments hitting a CpG counted the ISFs hitting a CpG with at least one end.

The number of distinct CpGs hits (out of 26567 annotated CpGs) by any fragment.

An Alu score giving the same information as the CPG score for overlaps with Alu repeats

The best predicted enzyme combination was found to be {MseI, Tsp509, NlaIII, HpyCH4} with an optimal length range of [346; 800], resulting in 99187 different in-slilico fragments (ISFs) of this length range per haploid male (X+Y) genome. In this case, almost all other (approx. 70 million) ISFs are shorter than 346nt, only 4964 ISFs are longer than 800nt. The selected enzyme combination is among the more robust ones in the sense that experimental inaccuracies in fragment length selection will not have detrimental effects. For example, the number of distinct fragments in the length range [330; 850] (roughly a 5% margin) under this combination is 123797 (24% increase), and considerably more for several other enzyme combinations. We find that 16312 (16.4%) of the ISFs hit a CpG; this constitutes a predicted 24-fold enrichment of CpGs in the selected ISFs, compared to random sampling. Overall, 12482 of the annotated 26567 distinct CpGs (47.0%) are hit by an ISF for the selected enzyme combination.

In practice, however, a worse CpG coverage than predicted is expected for several reasons: The in-silico study only takes into account those parts of chromosomes contained in the RefSeq assembly; this in particular excludes satellite repeat regions near centromers, making the statistics somewhat inaccurate. Fragment length selection in a gel cannot be expected to work perfectly. It is also expected that random fragment selection for sequencing is not unbiased.

To assess these adverse effects, the optimal predicted enzyme combination and length selection was applied to human genomic DNA. 389188 reads of average length 102nt were obtained using the 454 GS20 sequencing system, and mapped back to the genome using the VerJInxer software [available at http://verjinxer.googlecode.com]. It was found that 149141 reads (38%) could not be located according to the RefSeq genome; inspection revealed that they mainly consisted of satellite repeats. 15841 reads (4%) mapped to more than one position in the genome and could not be uniquely located. 120738 reads (31%) mapped uniquely to the approximate predicted regions (123797 distinct ISFs of lengths 330-850, accounting for about 5% inaccuracy in extracting fragments from the gel). The remaining 103468 reads (27%) mapped elsewhere in the genome.

Disregarding the non-predicted satellite repeat reads, about half of the remaining reads uniquely mapped to the predicted ISFs. Yet, the ISFs were not covered uniformly: 61879 ISFs were not hit by a read at all, 30672 once, 16365 twice, and further coverage frequencies continued similar to a geometric progression with factor 0.5. Concerning coverage of the 12482 predicted CpGs with the 120738 reads that could be mapped, it was found that 4754 CpGs were not covered, and 7728 CpGs were hit at least once. In detail, among these 7728 covered CpGs, 2894 were hit once, 1934 twice and 2900 more than twice.

Overall, 19238 of the mapped reads (15.9%; that is 4.94% of all 389188 reads) hit a CPG. This corresponded well with the prediction, at least among the reads that could be mapped. Unfortunately, however the adverse effects mentioned above result in less than a third of all reads mapping to the selected region. Nevertheless, compared to random genome sequencing, CpGs were enriched by a factor of 7.4.

As it will be shown below and in the examples section, the present invention is particularly advantageous when used in combination with the 454 Sequencing methodology (Margulies, M., Egholm, M., et al. Nature 437 (2005) 376-380). According to a specific embodiment of the present invention, a CG island enriched library is produced from genomic DNA by digestion with several restriction enzymes that preferably cut within non-CG island regions moreover, the restriction enzymes are selected in such a way that the digestion results in fragments with a size range between 350 to 800 bp. The library fragments are ligated to adaptors according to the conventional 454 single stranded library preparation workflow. Subsequently, a conventional Bisulfite treatment is performed according to methods that are well known in the Art: As a result, unmethylated cytosine residues are converted to Uracil residues, which in a subsequent sequencing reaction base calling are identified as "T" instead of "C", when compared with a non bisulfite treated reference. Subsequent to Bisulfite treatment, the sample is subjected to the conventional 454 sequencing protocol.

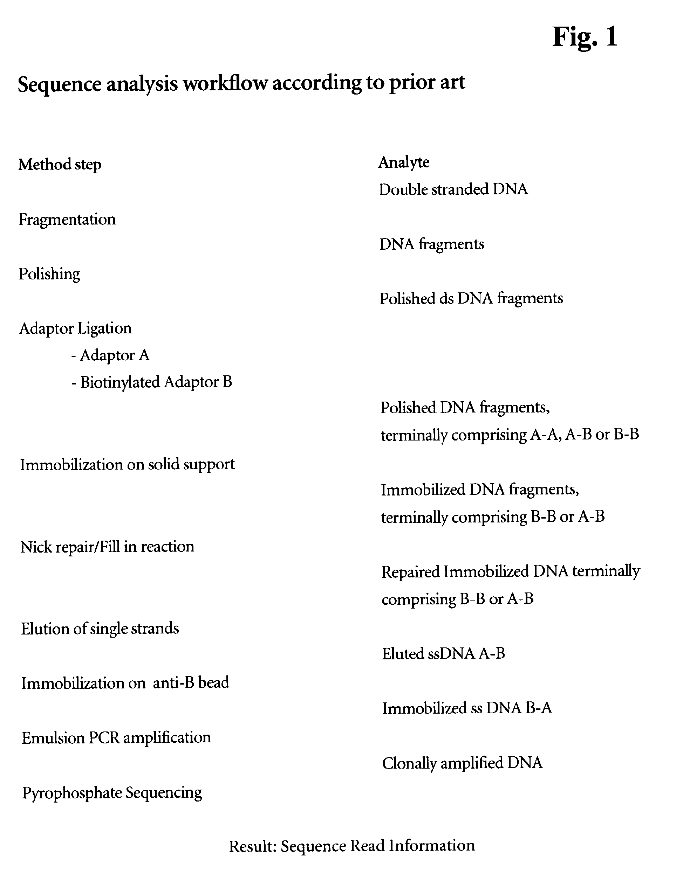

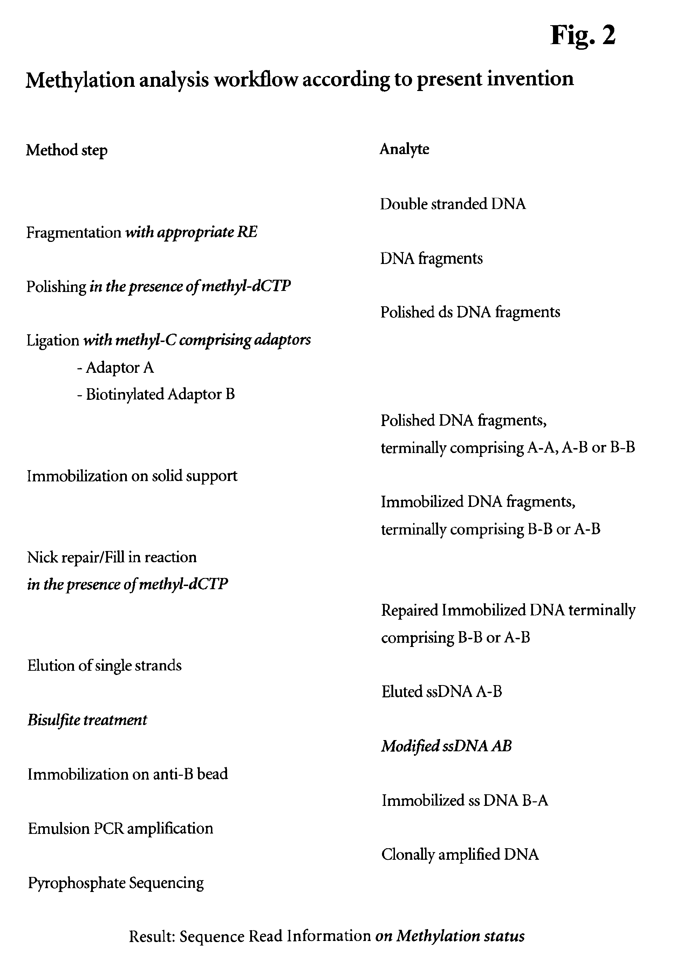

Fig. 1 is a schematic drawing showing the different steps of the 454 genome sequencer workflow. The improved method has been specifically modified for methylation analysis. It is shown by the schematic drawing of fig. 2. The separate steps can be defined as follows.

### - Sample Input and Fragmentation

The 454 Genome Sequencer System supports the sequencing of samples from a wide variety of starting materials including but not limited to eukaryotic or bacterial genomic DNA. Genomic DNAs are fractionated into small, 100- to 1000-bp fragments with an appropriate specific combination of restriction enzymes which enriches for CpG island containing fragments. In one embodiment, the restriction enzymes used for a method according to the present invention are selected from a group consisting of MseI, Tsp509, AluI, NlaIII, BfaI, HpyCH4, DpuI , MboII, MlyI, and BCCI, or any isoschizomer of any of the enzymes mentioned. Preferably, 4 - 5 different enzymes are selected. In a particular embodiment, 2 of the 4 - 5 different enzymes are MseI and Tsp509, or any isoschizomer of the two enzymes mentioned. Most preferably, a combination of MseI, Tsp509, NlaIII, and HpyCH4 or any respective isoschizomer is applied.

### - Library Preparation

Using a series of standard molecular biology techniques, short adaptors (A and B) - are added to each fragment. The adaptors are used for purification, amplification, and sequencing steps. Single-stranded fragments with A and B adaptors compose the sample library used for subsequent workflow steps.

Prior to ligation of the adaptors, the fragments have to be completely double stranded without any single stranded overhang. First, a fragment polishing reaction is performed using e.g. E.coli T4 DNA polymerase. In one embodiment, said polishing reaction is performed in the presence of Methyl-dCTP instead of dCTP. In an alternative embodiment, said fragment polishing reaction is performed in the presence of a DNA polymerase which lacks proofreading activity, such as Tth DNA polymerase (Roche Applied Science Cat. No: 11 480 014 001).

Then, the two different double stranded adaptors A and B (which is biotinylated) are ligated to the ends of the fragments. Preferably some or even all of the C-residues of adaptors A and B are Methyl-C residues. Subsequently, the fragments containing at least one B adaptor are immobilized on a Streptavidin coated solid support and a nick repair- fill-in synthesis is performed using a strand displacement enzyme such as Bst Polymerase (New England Biolabs). Preferably said reaction is performed in the presence of Methyl-dCTP instead of dCTP. Subsequently single stranded molecules comprising one adaptor A and one adaptor B are removed from the Streptavidin coated beads as disclosed in (Margulies, M., Egholm, M., et al., Nature 437 (2005) 376-380). In those case where Methy-dCTP replaces dCTP, it can be used at the same concentrations as dCTP is used in the original protocol.

### - Bisulfite treatment

The Bisulfite treatment can be done according to all standard method that are well known in the art (Frommer, M., et al.: "A genomic sequencing protocol that yields a positive display of 5-methylcytosine residues in individual DNA strands" Proc Natl Acad Sci USA 89 (1992) 1827-1831; Zeschnigk, M., et al., "Imprinted segments in the human genome: different DNA methylation patterns in the Prader-Willi/Angelman syndrome region as determined by the genomic sequencing method" Hum Mol Genet 6 (1997) 387-395; Clark, S.J., et al., " High sensitivity mapping of methylated cytosines" Nucleic Acids Res 22 (1994) 2990-2997). However, in many case it is highly advantageous, if subsequent to the treatment, the sample is purified, for example by an Sephadex size exclusion column or, at least by means of precipitation. It is also within the scope of the present invention, if directly after Bilsulfite treatment, or directly after Bilsulfite treatment followed by purification, the sample is amplified by means of performing a conventional PCR using amplification primers with sequences corresponding to the A and B adaptor sequences.

### - One Fragment = One Bead

The Bisulfite treated and optionally purified and/or amplified single-stranded DNA library is immobilized onto specifically designed DNA Capture Beads. Each bead carries a unique single-stranded DNA library fragment. The bead-bound library is emulsified with amplification reagents in a water-in-oil mixture resulting in microreactors containing just one bead with one unique sample-library fragment.

### - emPCR (Emulsion PCR) Amplification

Each unique sample library fragment is amplified within its own microreactor, excluding competing or contaminating sequences. Amplification of the entire fragment collection is done in parallel; for each fragment, this results in a copy number of several million per bead. Subsequently, the emulsion PCR is broken while the amplified fragments remain bound to their specific beads.

### - Sequencing: One Bead = One Read

The clonally amplified fragments are enriched and loaded onto a PicoTiterPlate device for sequencing. The diameter of the PicoTiterPlate wells allows for only one bead per well. After addition of sequencing enzymes, the fluidics subsystem of the Genome Sequencer FLX Instrument flows individual nucleotides in a fixed order across the hundreds of thousands of wells containing one bead each. Addition of one (or more) nucleotide(s) complementary to the template strand results in a chemiluminescent signal recorded by the CCD camera of the Genome Sequencer FLX Instrument.

### - Data Analysis

The combination of signal intensity and positional information generated across the PicoTiterPlate device allows the software to determine the sequence of more than 400,000 individual reads per 7.5-hour instrument run simultaneously. For sequencing-data analysis, a bioinformatics tools is available for resequencing of up to 3 gigabases. Comparison of the sequence data with the respective default sequences that are available in publicly accessible databases enable a discrimination between those C residues which were methylated in the original sample and those C residues that have been modified into Uracil residues and thus are identified by the sequence analysis as "T" residues.

### Description of the Figures

- Figure 1: Prior art sequencing workflow on the 454 Genome sequencer system
- Figure 2: Whole Genome methylation status analysis workflow according to the present invention. Differences to the state of the art workflow as depicted in fig. 1 are indicated by *italic* writing.

### Examples

### Example 1

### DNA isolation from blood and sperm

Genomic DNA was extracted and purified from blood and sperm cells using the FlexiGene DNA Kit (Qiagen, Hilden, Germany) following the manufacturer's instructions. Whole blood DNA was prepared from a female healthy donor. Semen samples were obtained from volunteers whose ejaculates were considered to be normal based on WHO criteria WHO: WHO Laboratory manual for the Examination of Human Semen and Sperm Cervical Mucus Interaction. Cambridge University press, 1999. Semen samples of volunteers were within the normal range with respect to sperm number (41 - 104 x 106 /ml) and all other tested parameter such as motility and morphology. DNA was quantified using the NanoDrop ND1000 instrument. For library generation and sequence analysis we pooled the DNA from 4 different semen samples.

### Example 2

### Preparation of DNA samples for massively parallel bisulfite pyrosequencing

Genomic DNA was isolated from spermatozoa and from blood cells from a female healthy donor. Said Genomic DNAs (30-40 µg) were digested using 30 - 50 U of each restriction enzymes MseI, Tsp509I, NlaIII and HpyCH4V, following the manufacturer's (NEB, Frankfurt, Germany) recommendations. Enzymes were applied sequentially to the DNA samples in the order listed above. After 2 h of incubation the enzyme activity was replenished by adding 30-50 U fresh enzyme to each sample and increasing the reaction volume accordingly with 1 x reaction buffer. Incubation was continued for another 2 h. After Tsp509I digestion, restriction fragments were phenol extracted followed by ethanol precipitation according to conventional methods known in the art. After final HpyCH4V digestion, DNA fragments were phenol extracted followed by ultra-filtration with Microcon 100 filtering devices at 500 x g. (Amicon). Typical yields were 10 µg of DNA. Whole amount of purified restriction fragments was size fractionated by electrophoresis on a 1.8 % agarose gel with 1 µg DNA applied per lane. Slices containing the 350-800 bp fractions were excised from the ethidium bromide stained gel and DNA was recovered by Wizard SV Gel and PCR Clean-Up System (Promega). DNA was concentrated by centrifugation in a Microcon 100 devices at 500 x g. Recovery from the gel was typically 400 ng.

### Example 3

### Library-Preparation for sequence analysis of bisulfite treated DNA fragments on GS FLX.

Library-Preparation of samples was performed according to the manufacturer's protocol "GS DNA Library Preparation Kit" (Margulies, M., Egholm, M., et al. Nature 437 (2005) 376-380) with the following modifications:

*Enzymatic polishing*. Without prior nebulization, restriction fragments were bluntended and phosphorylated through the activity of an enzyme mix (T4 DNA polymerase, *E. coli* DNA polymerase (Klenow fragment), T4 polynucleotide kinase) as described (Margulies, M., Egholm, M., et al. Nature 437 (2005) 376-380). The polishing reaction was performed in the presence of 5-methyl-dCTP (Jena Bioscience GmbH) instead of dCTP.

*Adaptor ligation*. The A and B adaptors each comprised of 44-base pair double stranded oligodeoxyribonucleotides were ligated to the polished ends of the DNA fragments. To preserve the adapter sequence in the following bisulfite modification step we used 5-methylcytosine-containing oligodeoxyribonucleotides. The 5' modification of the B adaptor with a biotin tag remains unaltered.

*Nick Repair-Fill-in synthesis*. The two nicks at the 3'-junctions were repaired by the strand-displacement activity of *Bst* DNA polymerase. For preservation of methylated cytosines in the adaptor sequence the reaction was performed in the presence of 5-methyl-dCTP (Jena Bioscience GmbH) instead of dCTP. Single stranded AB adapted library fragments were isolated as described in the manufacturer's protocol. DNA yield varied from 100 pg - 5 ng.

### Example 4

### Bisulfite modification, massively parallel pyrosequencing

Single stranded DNA fragments were subjected to bisulfite modification as described elsewhere (Zeschnigk, M., et al., Hum Mol Genet, 1997; 6:387-395), and finally resuspended in 10 µl of water. PCR amplification of 2 µl bisulphite converted library was performed in a total reaction volume of 25µl using GoTaq Green master Mix (Promega, USA) and 200nM of each of the primers GSFLXA and GSFLXB (Roche Applied Science Cat. 04 852 290 001). The primers GSFLXA and GSFLXB bind to 5 ' part of the adaptors A and B, respectively. After initial denaturation at 95°C for 10 min, 35 cycles were carried out with denaturation at 95°C for 30 sec annealing at 62°C for 30 sec and extension at 72°C for 1 min, followed by a final extension at 72°C for 5 min. To remove unspecific reaction products gel purification of PCR products was performed as described above, however, DNA fragments in the size of 440-900 bp were recovered. The DNA was quantified using the Agilent 2100 BioAnalyzer on a RNA 6000 Pico LabChip.

Bead preparation and sequence analysis was performed according to the Shotgun protocol of the GS emPCR Kit User's Manual (Roche Applied Science Cat. 04 852 290 001) and Genome Sequencer FLX". Massively parallel pyrosequencing of the amplification products was executed according to the manufacturer's provided protocol (Roche Applied Science Cat. No. 04 9312 315 001).

### Example 5

### Data analysis

For the determination of methylation status, the reads were mapped against the CpG islands of a respective Reference Sequence (CpG island definition according to UCSC (Gardiner-Garden, M., and Frommer, M., J Mol Biol 196 (1987) 261-282).

A total number of 163034 and 129621 reads from the blood and sperm libraries were obtained, respectively, with an average read length of 134 bp. Bioinformatic analysis, revealed that 12358 (7,8 %) blood library reads map to 6167 individual CpGs, from which 1409 (23%) CpGs were found, at least in part, to be methylated. From the sperm library 10216 (7,4 %) reads map to 5796 individual CpGs. However, in this sample only 820 (14%) of all CPG were found to be methylated. Differentially methylated CpGs, which are indicated by the presence of reads from methylated and unmethylated fragments, were observed in 63 and 53 CpGs in the blood and sperm library, respectively. Significant differences between sperm and blood samples were found on the X chromosome as only 2 methylated CpGs were identified in sperm. We exemplarily verified the results of 8 fully methylated and 3 differentially methylated CpGs by conventional bisulfite sequencing and found complete concordance.

## Claims

1. Method for analyzing the methylation status of a genomic DNA sample, comprising the steps of
a) fragmenting said sample and enriching said sample for sequences comprising CpG islands
b) generating a single stranded DNA library
c) subjecting said sample to Bisulfite treatment
d) clonally amplifying individual members of said single stranded DNA library by means of emulsion PCR
e) sequencing said amplified clonally amplified single stranded DNA library.

2. Method according to claim 1, **characterized in that** the methylation status of at least 1000 and preferably 10 000 CpG islands is analyzed simultaneously.

3. Method according to claims 1-2, wherein step a) comprises the steps of
- digesting said sample with a plurality of restriction enzymes which more frequently cut regions of DNA comprising no CpG islands and less frequently cut regions comprising CpG islands
- isolating DNA fragments with a specific size range.

4. Method according to claim 3, wherein said size range has a lower limit of 100, preferably 200 and most preferably 300 base pairs and an upper limit of 1000, preferably 900 and most preferably 800 base pairs.

5. Method according to claims 3-4, wherein said restriction enzymes are selected from a group consisting of MseI, Tsp509, AluI, NlaIII, BfaI, HpyCH4, DpuI, MboII, Mlyl, BCCI and any isoschizomer thereof.

6. Method according to claims 1-5, wherein step b) comprises the steps of
- performing a fragment polishing reaction
- performing a ligation reaction comprising two different double stranded adaptors A and B
- selecting for single stranded molecules comprising one adaptor A and one adaptor B.

7. Method according to claim 6, wherein the fragment polishing reaction is performed in the presence of 5-Methyl-dCTP.

8. Method according to claims 6-7, wherein the C-residues of adaptors A and B are 5-Methyl-C residues.

9. Method according to claims 6-8, **characterized in that** between said steps of performing a ligation reaction and selecting for single stranded molecules comprising one adaptor A and one adaptor B, a nick repair-fill-in reaction is performed in the presence of 5-Methyl-dCTP.
